# EUROPÄISCHE PATENTANMELDUNG

(11) **EP 4 623 945 A1**
(43) Veröffentlichungstag der Anmeldung: **01.10.2025**
(21) Anmeldenummer: 25165342.4
(22) Anmeldetag: 21.03.2025
(51) Int. Cl.: A61M 1/16

(54) **BEHÄLTER-ANORDNUNG ZUR HERSTELLUNG UND BEREITSTELLUNG EINER ALKALISIERENDEN LÖSUNG, SOWIE EXTRAKORPORALE BLUTBEHANDLUNGSMASCHINE MIT DER BEHÄLTER-ANORDNUNG**

(30) Priorität: 26.03.2024 DE 102024108582
(71) Anmelder: B. Braun Avitum AG, 34212 Melsungen (DE)
(72) Erfinder: HAGE, Thorsten, 49186 Bad Iburg (DE); FIEBIG, Dinah, 48147 Münster (DE)
(74) Vertreter: Winter, Brandl - Partnerschaft mbB

(57) **Zusammenfassung**

Die Offenbarung betrifft eine Behälter-Anordnung (58) für eine oder einer extrakorporalen Blutbehandlungsmaschine (1) als Ersatz eines mit vorbestimmter Topologie ausgestalteten Einsatz-Behälters, zur Bereitstellung einer alkalisierenden Lösung aufweisend: einen Behälter (60), der vorgesehen und ausgebildet ist, alkalisierende Trockensubstanz aufzunehmen, mit einem Behälter-Einlauf (62) und einem Behälter-Auslauf (64), einen Adapter (66) mit einem Zulauf-Verbindungsabschnitt (70), der zur Kopplung und fluidischen Verbindung mit einem Zulauf (68) einer Einsatz-Behälter-Aufnahme (68, 72) der extrakorporalen Blutbehandlungsmaschine (1) vorgesehen und ausgestaltet ist, und einem Ablauf-Verbindungsabschnitt (74), der zumindest zur Kopplung mit einem Ablauf (72) der Einsatz-Behälter-Aufnahme (68, 72) der extrakorporalen Blutbehandlungsmaschine (1) vorgesehen und ausgestaltet ist, und einen Lösungsmittel-Strömungspfad (82), der von dem Zulauf-Verbindungsabschnitt (70) des Adapters (66), über den Behälter-Einlauf (62), durch den Behälter (60) und zu dem Behälter-Auslauf (64) vorgesehen und ausbildbar ist. Darüber hinaus betrifft die Offenbarung eine extrakorporale Blutbehandlungsmaschine mit einer Behälter-Anordnung.

## Beschreibung

### Technisches Gebiet

Die vorliegende Offenbarung betrifft eine Behälter-Anordnung zur Herstellung und Bereitstellung einer alkalisierenden Lösung aus alkalisierender Trockensubstanz für eine extrakorporale Blutbehandlungsmaschine, sowie eine extrakorporale Blutbehandlungsmaschine, insbesondere eine Dialysemaschine, für eine extrakorporale Blutbehandlung, wie etwa eine Hämodialyse, eine Hämofiltration, eine Hämodiafiltration und/oder eine Ultrafiltration. Die Blutbehandlungsmaschine hat einen Dialysator mit einer semipermeablen Membran für einen Stoffaustausch zwischen dem in einem extrakorporalen Blutkreislauf geführten Blut eines Patienten und einer Dialysierflüssigkeit eines Dialysierflüssigkeitskreislaufs, welcher über einen Bluteingang und einen Blutausgang des Dialysators durch den Dialysator verläuft. Von einer Mischeinheit der Blutbehandlungsmaschine wird Reinstwasser mit einer alkalisierenden Trockensubstanz, insbesondere mit einer Bicarbonat-Trockensubstanz, gemischt, insbesondere um eine mögliche Azidose zu verhindern. Ergänzend wird in der Mischeinheit ein saures Konzentrat beigemischt. Dem Dialysierflüssigkeitskreislauf wird in Folge die so hergestellte, frische Dialysierflüssigkeit bereitstellt.

### Technischer Hintergrund

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen und über einen extrakorporalen Blutkreislauf einem Dialysator für eine Blutbehandlung zugeführt. Dem Dialysator wird zudem bedarfsgerecht hergestellte, frische Dialysierflüssigkeit über einen Dialysierflüssigkeitskreislauf zugeführt. Zur Herstellung der frischen Dialysierflüssigkeit wird von einer Wasseraufbereitung, insbesondere von einer Umkehrosmoseanlage, Reinstwasser bereitgestellt, entgast und in Folge in einer Mischeinheit mit einer alkalisierenden Trockensubstanz und einem sauren Konzentrat gemischt. Als alkalisierende Trockensubstanz findet beispielsweise Bicarbonat Verwendung. Das Bicarbonat wird im Falle einer intermittierenden oder ambulanten Blutbehandlung, insbesondere bei der Therapie einer chronischen Niereninsuffizienz, in einem Einsatz-Behälter bereitgestellt, der eine auf die Blutbehandlungsmaschine abgestimmte, vorbestimmte Größe und Anschluss-Topologie aufweist. An der Blutbehandlungsmaschine ist entsprechend eine Einsatz-Behälter-Aufnahme vorbestimmter Größe und Anschluss-Topologie vorgesehen. Der Einsatz-Behälter wird in diese speziell auf ihn abgestimmte Einsatz-Behälter-Aufnahme eingesetzt. Über eine Sensorik wird erfasst, ob tatsächlich beide Anschlüsse der Einsatz-Behälter-Aufnahme - das heißt, ein Zulaufanschluss, an dem das Reinstwasser ansteht, und ein Ablaufanschluss, an dem die gemischte Bicarbonat-Lösung bereitgestellt wird - mit der Aufnahme gekoppelt sind und fluidisch verbunden sind. So wird erfasst, ob die Einsatz-Behälter-Aufnahme wirklich mit dem vorbestimmten Einsatz-Behälter besetzt ist und die notwendige Fluidverbindung des Zu- und Ablaufs korrekt ausgebildet ist, sodass das Lösen der Bicarbonat-Trockensubstanz prozesssicher und bestimmungsgemäß erfolgen kann. Wird die Einsatz-Behälter-Aufnahme als korrekt besetzt erfasst, erfolgt über eine Steuereinheit die Freigabe des Mischbetriebs, welcher selbst eine Voraussetzung für die Blutbehandlung darstellt.

Herkömmliche Einsatz-Behälter, wie beispielsweise die auf die Anmelderin zurückgehende und unter dem Namen Sol-Cart^{®} B bekannte Bicarbonat-Kartusche oder der unter dem Namen bibag^{®} bekannte Konzentratbeutel, enthalten eine vergleichsweise geringe Menge an Trockensubstanz und unterstützen stets nur die einmalige Durchführung der Blutbehandlung.

Nachteilig an diesen Lösungen ist, dass die geringe Menge ein häufiges Umrüsten/ Auswechseln des Einsatz-Behälters erforderlich macht. Zudem sind diese Einsatz-Behälter nur zum einmaligen Gebrauch (single use) bestimmt und nicht benötigte Restmengen müssen verworfen werden. Damit ist ein Materialeinsatz an primären Verpackungsmaterialien, insbesondere des verwendeten Kunststoffes für den Einsatz-Behälter, wie beispielsweise PET, PE oder PP, hoch, was eine Umweltkompatibilität/ - bilanz negativ beeinflusst.

### Zusammenfassung der vorliegenden Offenbarung

Die Aufgabe der vorliegenden Offenbarung ist demgegenüber, die Nachteile aus dem Stand der Technik zu vermeiden oder zumindest zu mindern und insbesondere eine Behälter-Anordnung, sowie eine extrakorporale Blutbehandlungsmaschine zur Verfügung zu stellen, welche eine effizientere und sicherere, intermittierende oder ambulante, extrakorporale Blutbehandlung bereitstellt.

Die Aufgabe der vorliegenden Offenbarung wird hinsichtlich einer Behälter-Anordnung offenbarungsgemäß durch die Merkmale des Anspruchs 1 und hinsichtlich der extrakorporalen Blutbehandlungsmaschine offenbarungsgemäß durch die Merkmale des Anspruchs 9 gelöst. Weiterbildungen sind in den abhängigen Ansprüchen beschrieben.

Ein Grundgedanke der vorliegenden Offenbarung sieht zur Bereitstellung einer alkalisierenden Trockensubstanz und zur Herstellung und Bereitstellung von einer alkalisierenden Lösung vor, in eine mit vorbestimmter mechanischer und fluidischer Anschluss-Topologie ausgestaltete Einsatz-Behälter-Aufnahme einer extrakorporalen Blutbehandlungsmaschine, anstatt eines gemäß dieser Topologie vorbestimmt ausgestalteten Einsatz-Behälters, einen gemäß dieser Topologie angepasst ausgestalteten Adapter einzusetzen, mechanisch zu koppeln und fluidisch zu verbinden. Dabei kann der Adapter bezüglich seines Volumens, verglichen mit dem vorbestimmten Einsatz-Behälter, kleinere Abmessungen aufweisen. An diesen Adapter ist offenbarungsgemäß ein von der Topologie der Einsatz-Behälter-Aufnahme unabhängig ausgestalteter, die alkalisierende Trockensubstanz beinhaltender Behälter fluidisch angeschlossen. Der Behälter kann insbesondere entfernt von der Einsatz-Behälter-Aufnahme an der extrakorporalen Blutbehandlungsmaschine vorgesehen sein.

Es ergibt sich der Vorteil, dass zumindest der Lösungsmittel-Zulauf, und vorzugsweise auch der Lösungs-Ablauf der Einsatz-Behälter-Aufnahme, der Blutbehandlungsmaschine vom Adapter zur Zufuhr von Lösungsmittel/ Reinstwasser und vorzugsweise zur Bereitstellung der alkalisierenden Lösung genutzt werden kann, und dass die vom Behälter bereitgestellte Menge an Trockensubstanz nicht mehr auf die vergleichsweise kleine Menge des vorbestimmt ausgestalteten Einsatz-Behälters beschränkt ist. Vorzugsweise ist der Behälter der offenbarungsgemäßen Behälter-Anordnung zur Verwendung über mehrere Blutbehandlungen hinweg ausgelegt und ausgestaltet, insbesondere bezüglich seines Materials und seiner Inhaltsmenge. Mit diesem Behälter, der vorzugsweise eine größere Menge Trockensubstanz beinhaltet als der vorbestimmte Einsatz-Behälter, können somit an derselben Blutbehandlungsmaschine in sequentieller Abfolge mehrere Blutbehandlungen durchgeführt werden, man kann auch sagen kontinuierlich durchgeführt werden, ohne dass der Behälter gewechselt werden muss. Im Vergleich zum vorbestimmten, vergleichsweise kleineren Einsatz-Behälter muss der über den Adapter fluidisch verbundene, vergleichsweise größere Behälter somit weniger oft gewechselt werden, was zu einem reduzierten Aufwand während der Blutbehandlungen und zu einer Kostenreduktion im Herstellungsprozess führt, da für eine vorbestimmte Menge Trockensubstanz weniger Behälter/ Behältermaterial benötigt werden/ wird. Hinzu kommt, dass - anders als bei dem für den Einmalgebrauch (single-use) vorgesehenen Einsatz-Behälter - kein regelmäßiger Verwurf von Restmengen erfolgt. Die Einsatz-Behälter-Aufnahme kann selbstverständlich, alternativ zur offenbarungsgemäßen Lösung, weiterhin mit dem vorbestimmten Einsatz-Behälter im Einmalgebrauch (single-use) genutzt werden.

In anderen Worten ausgedrückt, besteht ein der vorliegenden Offenbarung zugrundeliegender Gedanke darin, einer bestehenden/ bekannten extrakorporalen Blutbehandlungsmaschine, anstatt eines vorbestimmten Einsatz-Behälters - welcher insbesondere bezüglich seiner beinhalteten Menge Trockensubstanz und seiner Anschluss-Topologie und Anschluss-Abmessung, insbesondere einer Topologie und Abmessung seines fluidischen Zulaufs und Ablaufs, vorbestimmt ausgestaltet ist - einen Adapter mit gleicher, vorbestimmter Anschluss-Topologie und Anschluss-Abmessung bereitzustellen und mit dem Adapter einen Behälter mit - gegenüber dem vorbestimmten Einsatz-Behälter vorzugsweise größerer Menge - Trockensubstanz fluidisch zu verbinden. Der offenbarungsgemäße Adapter ist vorzugsweise vorgesehen und ausgestaltet, in eine für den vorbestimmt ausgestalteten Einsatz-Behälter vorgesehene, vorbestimmt ausgestaltete Einsatz-Behälter-Aufnahme der bestehenden/ bekannten extrakorporalen Blutbehandlungsmaschine eingesetzt zu werden. So kann offenbarungsgemäß einerseits die Bereitstellung von Lösungsmittel, vorzugsweise von Reinstwasser, über die vorbestimmt ausgestaltete Einsatz-Behälter-Aufnahme erfolgen, und andererseits kann ein die Trockensubstanz beinhaltender Behälter angeschlossen/ verwendet werden, der bezüglich seiner beinhalteten Menge, seiner Anschluss-Topologie und seiner Abmessungen, insbesondere der Topologie und Abmessungen seines fluidischen Zulaufs und Ablaufs, frei wählbar ausgestaltet ist.

**In** nochmals anderen Worten wird gemäß der vorliegenden Offenbarung eine Behälter-Anordnung für eine oder einer extrakorporalen Blutbehandlungsmaschine bereitgestellt, die zur intermittierenden oder ambulanten Blutbehandlung vorgesehen und ausgestaltet ist. Die offenbarungsgemäße Behälter-Anordnung ist dabei als Ersatz für einen mit vorbestimmter Topologie ausgestalteten Einsatz-Behälter zur Bereitstellung einer alkalisierenden Trockensubstanz, zur Lösung der Trockensubstanz und zur Bereitstellung der resultierenden, alkalisierenden Lösung, insbesondere einer Bicarbonat-Lösung, vorgesehen und ausgestaltet. Die Behälter-Anordnung weist hierzu auf:
einen Behälter, der vorgesehen und ausgebildet ist, die alkalisierende Trockensubstanz aufzunehmen, mit einem Lösungsmittel in Kontakt zu bringen und die alkalisierende Lösung bereitzustellen, wobei der Behälter einen Behälter-Einlauf, der zur fluidischen Verbindung mit einem Lösungsmittel-Zulauf der Blutbehandlungsmaschine vorgesehen und ausgebildet ist, und einen Behälter-Auslauf, der zur Bereitstellung der alkalisierenden Lösung, insbesondere an einen Dialysierflüssigkeitskreislauf der extrakorporale Blutbehandlungsmaschine, vorgesehen und ausgebildet ist, hat;
einen, insbesondere fluidischen, Adapter, der vorgesehen und ausgebildet ist, als ein Ersatz für den Einsatz-Behälter in eine vorbestimmt ausgestaltete Einsatz-Behälter-Aufnahme der extrakorporalen Blutbehandlungsmaschine eingesetzt zu werden. Vorzugsweise ist die Einsatz-Behälter-Aufnahme zur Kopplung und fluidischen Verbindung mit dem Einsatz-Behälter vorgesehen und vorbestimmt ausgestaltet. Hierfür hat die Einsatz-Behälter-Aufnahme vorzugsweise einen fluidisch mit dem Lösungsmittel-Zulauf der extrakorporalen Blutbehandlungsmaschine verbindbaren, insbesondere verbundenen, Zulauf und einen fluidisch mit dem Dialysierflüssigkeitskreislauf der extrakorporalen Blutbehandlungsmaschine verbindbaren, insbesondere verbundenen, Ablauf. Der Zulauf und der Ablauf der Einsatz-Behälter-Aufnahme sind vorzugsweise vorbestimmt zueinander angeordnet und ausgebildet. Insbesondere sind sie gemäß der vorbestimmten Topologie des Einsatz-Behälters vorbestimmt zueinander angeordnet und ausgebildet. Der Adapter hat offenbarungsgemäß einen Zulauf-Verbindungsabschnitt, der zur Kopplung und fluidischen Verbindung mit dem Zulauf der Einsatz-Behälter-Aufnahme der extrakorporalen Blutbehandlungsmaschine vorgesehen und ausgestaltet ist, und einen Ablauf-Verbindungsabschnitt, der zumindest zur Kopplung - das heißt, zur Positionierung und/ oder Befestigung, jedoch nicht unbedingt auch zur fluidischen Verbindung - mit dem Ablauf der Einsatz-Behälter-Aufnahme der extrakorporalen Blutbehandlungsmaschine vorgesehen und ausgestaltet ist;
sowie zumindest einen Lösungsmittel-Strömungspfad, vorzugsweise eine Lösungsmittel-Leitung und/ oder einen Lösungsmittel-Schlauch, der von dem Zulauf-Verbindungsabschnitt des Adapters, über den Behälter-Einlauf, durch den Behälter und zu dem Behälter-Auslauf vorgesehen und ausbildbar ist.

Wie weiter oben bereits erwähnt, ist es mittels der offenbarungsgemäßen Behälter-Anordnung möglich, die vorbestimmt ausgestaltete Einsatz-Behälter-Aufnahme einer extrakorporalen Blutbehandlungsmaschine zur fluidischen Anbindung eines Behälters zu nutzen, welcher die Topologie des vorbestimmten Einsatz-Behälters nicht erfüllt/ nicht erfüllen muss. Mit Hilfe der offenbarungsgemäßen Behälter-Anordnung sind also unterschiedliche Behälter, unterschiedlicher Topologie, insbesondere unterschiedlicher Anschluss-Topologie, mit der Einsatz-Behälter-Aufnahme koppelbar und fluidisch verbindbar. Das ist darin begründet, dass nicht der Behälter in die Einsatz-Behälter-Aufnahme eingesetzt und gekoppelt wird, sondern der Adapter der Behälter-Anordnung, welcher die von der Einsatz-Behälter-Anordnung verlangte Topologie, insbesondere Anschluss-Topologie, aufweist. Es kann somit ein Behälter beliebiger Topologie und beliebiger Inhaltsmenge fluidisch verbunden werden. Wie oben erwähnt ist der vorbestimmte Einsatz-Behälter zum einmaligen Gebrauch (single use) bestimmt. Nicht benötigte Restmengen der beinhalteten Trockensubstanz müssen dann stets verworfen werden. Mit der offenbarungsgemäßen Behälter-Anordnung können selbst Großgebinde als Behälter angeschlossen werden, die bedeutend mehr Trockensubstanz beinhalten als der vorbestimmte Einsatz-Behälter. Das wiederum ergänzt den Einsatz des vorbestimmten Einsatz-Behälters an der extrakorporalen Blutbehandlungsmaschine um Behälter beliebiger Größe und Topologie, sodass der angeschlossene Behälter über die einmalige Durchführung der Blutbehandlung/ Dialysetherapie hinausgehend genutzt werden kann. Das häufige Umrüsten/ Auswechseln, das bei Verwendung des vergleichsweise kleinen, vorbestimmten Einsatz-Behälters unabhängig von seinem Restfüllstand nach einer jeweiligen Therapie nötig ist, wird reduziert, bzw. ist nicht mehr erforderlich. Mittels der offenbarungsgemäßen Behälter-Anordnung sind somit bei entsprechend groß gewähltem Behälter, beispielsweise mit einem Inhalt von mehr als einem Liter, insbesondere zwei bis fünf Litern Trockensubstanz, Mehrfachanwendungen möglich und der Inhalt kann über mehrere Blutbehandlungen hinweg verwendet werden. Ein Überschuss aus einer ersten Blutbehandlung steht so noch für eine folgende Blutbehandlung zur Verfügung und muss nicht verworfen werden. Erst nach Verbrauch der Trockensubstanz des Behälters muss an den Adapter ein neuer Behälter mit alkalisierender Trockensubstanz angeschlossen werden. Auf diese Weise reduziert sich der Materialeinsatz an primären Verpackungsmaterialien, insbesondere des verwendeten Kunststoffes für den Behälter, wie beispielsweise PET, PE oder PP, deutlich. Hierdurch wird auch die Umweltkompatibilität/-bilanz des Produktes deutlich verbessert.

Kurz gesagt ist offenbarungsgemäß eine Behälter-Anordnung zur Bereitstellung von alkalisierender Trockensubstanz und deren Lösung bereitgestellt, durch die eine intermittierende oder ambulante, extrakorporale Blutbehandlung effizienter und sicherer erfolgen kann.

Vorzugsweise ist der Behälter/ die beinhaltete Menge Trockensubstanz so bemessen, dass mit der Blutbehandlungsmaschine die Blutbehandlung über eine Woche kontinuierlich erfolgen kann. Hierbei liegt ein Volumen des Behälters/ der Menge beinhalteter Trockensubstanz bei mehr als einem Liter, insbesondere bei 1,5 bis 5 Litern.

Dadurch, dass der Behälter topologisch nicht (mehr) in die vorbestimmte Einsatz-Behälter-Aufnahme passen muss, ist er vorzugsweise bezüglich seiner Lagerfähigkeit und/oder seines Transports optimiert. Vorzugsweise weist er eine quaderförmige, insbesondere stapelbare Grundform auf.

Die alkalisierende Trockensubstanz ist vorzugsweise ein pharmazeutisches Feststoffkonzentrat, vorzugsweise ein Dialysekonzentrat, basierend auf einem Bicarbonat, vorzugsweise Natriumbicarbonat, zur dynamischen Mehrfachanwendung im Zuge einer Dialysetherapie.

Gemäß einer möglichen Weiterbildung der Behälter-Anordnung ist zur Bereitstellung der in dem Behälter durch Lösen der Trockensubstanz hergestellten, alkalisierenden Lösung an den Dialysierflüssigkeitskreislauf der extrakorporalen Blutbehandlungsmaschine ein erster Bereitstellungs-Strömungspfad von dem Behälter-Auslauf ausgehend und unter Umgehung des Ablauf-Anschlusses des Adapters vorgesehen. Auf diese Weise kann die Anbindung des Behälter-Auslaufs unabhängig von dem Ablauf-Anschluss des Adapters und damit unabhängig von dem Ablauf der Einsatz-Behälter-Aufnahme erfolgen. Die Bereitstellung kann so sehr flexibel, beispielsweise an einen Lösungs-Container, erfolgen.

Vorzugsweise mündet/ endet der erste Bereitstellungs-Strömungspfad an einer Entnahmelanze, die in einen Lösungs-Container der extrakorporalen Blutbehandlungsmaschine einsetzbar/ eintauchbar ist.

In alternativer oder ergänzender Weiterbildung der Behälter-Anordnung ist zur Bereitstellung der in dem Behälter durch Lösen der Trockensubstanz hergestellten, alkalisierenden Lösung an den Dialysierflüssigkeitskreislauf der extrakorporalen Blutbehandlungsmaschine ein zweiter Bereitstellungs-Strömungspfad von dem Behälter-Auslauf ausgehend zu dem Ablauf-Anschluss des Adapters vorgesehen. Auf diese Weise kann die Anbindung des Behälter-Auslaufs über den Ablauf-Anschluss des Adapters und unter Nutzung des Ablaufs der Einsatz-Behälter-Aufnahme erfolgen. Die Bereitstellung kann so über die vorbestimmte fluidische Verbindung des Ablaufs der Einsatz-Behälter-Aufnahme der extrakorporalen Blutbehandlungsmaschine erfolgen.

Gemäß einer bevorzugten Weiterbildung weist die Behälter-Anordnung einen, vorzugsweise schaltbaren, Bypass-Strömungspfad auf, der unter Umgehung des Behälters von dem Lösungsmittel-Anschluss des Adapters zu dem Ablauf-Anschluss des Adapters vorgesehen ist. Der Bypass-Strömungspfad verläuft vorzugsweise innerhalb des Adapters, vorzugsweise als Schlauch- oder Rohrverbindung. Auf diese Weise ist der die Trockensubstanz beinhaltende Behälter der Behälter-Anordnung jederzeit in den Bypass schaltbar, bzw. umgehbar, und der Adapter ermöglicht eine Desinfektion der Leitungen und Schläuche der extrakorporalen Blutbehandlungsmaschine, ohne dass der Behälter entnommen oder abgebaut werden muss. Trotz der vorzugsweise großen Menge Trockensubstanz des Behälters kann auf diese Weise jederzeit desinfiziert werden, ohne den Behälter entnehmen oder abbauen zu müssen.

Um zumindest den oben genannten Lösungsmittel-Strömungspfad schalten zu können, weist die Behälter-Anordnung in einer bevorzugten Weiterbildung stromabwärts des Zulauf-Verbindungsabschnitts ein erstes Wegeventil mit Schaltstellungen auf. Dabei ist der Lösungsmittel-Strömungspfad in einer ersten Schaltstellung des ersten Wegeventils aufgesteuert und in einer zweiten Schaltstellung des ersten Wegeventils gesperrt. In einfachster Ausführung ist das erste Wegeventil als manuell betätigbares 2/2-Wegeventil mit zwei Anschlüssen und zwei Schaltstellungen ausgebildet, wobei einer der Anschlüsse mit dem Zulauf-Verbindungsabschnitt und der andere mit dem Behälter-Einlauf fluidisch verbunden ist.

Um den oben genannten zweiten Bereitstellungs-Strömungspfad über den Behälter-Auslauf zum Ablauf-Verbindungsabschnitt des Adapters schalten zu können, weist die Behälter-Anordnung in einer bevorzugten Weiterbildung stromaufwärts des Ablauf-Verbindungsabschnitt des Adapters ein zweites Wegeventil mit Schaltstellungen auf. Dabei ist der zweite Bereitstellungs-Strömungspfad in einer ersten Schaltstellung des zweiten Wegeventils aufgesteuert und in einer zweiten Schaltstellung des zweiten Wegeventils gesperrt. In einfachster Ausführung ist auch das zweite Wegeventil als manuell betätigbares 2/2-Wegeventil mit zwei Anschlüssen und zwei Schaltstellungen ausgebildet, wobei einer der Anschlüsse mit dem Behälter-Auslauf und der andere mit dem Ablauf-Verbindungsabschnitt des Adapters fluidisch verbunden ist.

Im Falle der Weiterbildung mit dem Bypass-Strömungspfad ist dieser vorzugsweise mittels dem ersten und dem zweiten Wegeventil schaltbar. Vorzugsweise ist dabei der Bypass-Strömungspfad mit den ersten Schaltstellungen der beiden Wegeventile gesperrt und mit den zweiten Schaltstellungen der beiden Wegeventile aufgesteuert. In diesem Fall sind die Wegeventile in einfacher Ausführung vorzugsweise als manuell betätigbare 3/2-Wegeventile mit drei Anschlüssen und zwei Schaltstellungen ausgebildet. Die 3/2-Wegeventile weisen dabei die gleichen Anschlüsse und Verbindungen wie die beiden oben genannten 2/2-Wegeventile auf, sind darüber hinaus aber jeweils um einen dritten Anschluss ergänzt, wobei die dritten Anschlüsse miteinander über den Bypass-Strömungspfad verbunden sind.

Innerhalb des Behälters kann der Lösungsmittel-Strömungspfad unterschiedlich geführt sein, was in unterschiedlichen baulichen Ausführungen oder Grundkonzepten des Behälters resultiert.

Gemäß einer ersten Weiterbildung des Behälters ist sowohl der Behälter-Einlauf, an dem ein Eintritt des Lösungsmittels vorgesehen ist, als auch der Behälter-Auslauf, an dem der Austritt der alkalisierenden Lösung vorgesehen ist, am höchsten Punkt oder Bereich des Behälters vorgesehen. Hierzu erstreckt sich vom Behälter-Auslauf ein Tauchrohr in den Behälter und mündet im tiefsten Punkt oder Bereich des Behälters. An dieser Mündung weist das Tauchrohr vorzugsweise einen Filter auf, um den Eintritt ungelöster Trockensubstanz zu verhindern. Das Lösungsmittel tritt somit oben in den Behälter ein und strömt/ sickert durch die Trockensubstanz und löst diese, sodass am tiefsten Punkt oder Bereich des Behälters die alkalisierende Lösung in das Tauchrohr eintritt und, insbesondere aufgrund des fortwährend nachlaufenden Lösungsmittels, zum Behälter-Auslauf verdrängt wird.

In alternativer Ausgestaltung ist der Behälter-Einlauf am höchsten Punkt oder Bereich des Behälters und der Behälter-Auslauf ist randseitig am Behälter vorgesehen, wobei sich auch in diesem Fall vom Behälter-Auslauf ein Tauchrohr in den Behälter erstreckt und im tiefsten Punkt oder Bereich des Behälters mündet. Auch in diesem Fall weist die Mündung des Tauchrohres vorzugsweise einen Filter auf, um den Eintritt ungelöster Trockensubstanz ins Tauchrohr zu verhindern.

In alternativer Ausgestaltung ist der Behälter-Einlauf am höchsten Punkt oder Bereich des Behälters vorgesehen und der Behälter-Auslauf ist am tiefsten Punkt oder Bereich des Behälters vorgesehen. In diesem Fall ist dem Behälter-Auslauf vorzugsweise ein Filter vorgelagert, um den Austritt ungelöster Trockensubstanz zu verhindern.

Eine extrakorporale Blutbehandlungsmaschine, insbesondere Dialysemaschine, ist gemäß der vorliegenden Offenbarung zur intermittierenden oder ambulanten extrakorporalen Blutbehandlung von Blut eines Patienten vorgesehen und ausgebildet. Offenbarungsgemäß weist sie auf:
einen Dialysator;
einen Dialysierflüssigkeitskreislauf, welcher über einen Dialysierflüssigkeitseingang und einen Dialysatausgang des Dialysators durch den Dialysator verläuft;
eine Mischeinheit, die dafür vorgesehen und angepasst ist, zumindest Reinstwasser und eine alkalisierende Trockensubstanz, vorzugsweise eine Bicarbonat-Trockensubstanz, vorzugsweise ein Natrium-Bicarbonat, zu einer alkalisierenden Lösung zu mischen/ zu lösen und die alkalisierende Lösung dem Dialysierflüssigkeitskreislauf bereitzustellen;
eine Einsatz-Behälter-Aufnahme der Mischeinheit, die gemäß wenigstens einem Aspekt der vorangegangenen Beschreibung ausgestaltet ist, und die zur Kopplung und fluidischen Verbindung mit einem mit vorbestimmter Topologie ausgestalteten Einsatz-Behälter zur Bereitstellung der alkalisierenden Lösung vorgesehen und ausgestaltet ist, wobei die Einsatz-Behälter-Aufnahme einen Zulauf hat, der mit einem Lösungsmittel-Zulauf der extrakorporalen Blutbehandlungsmaschine fluidisch verbindbar, insbesondere verbunden, ist, und einen Ablauf hat, der zur Bereitstellung der alkalisierenden Lösung mit dem Dialysierflüssigkeitskreislauf fluidisch verbindbar, insbesondere verbunden, ist. Der Zulauf und der Ablauf der Einsatz-Behälter-Aufnahme sind offenbarungsgemäß vorbestimmt zueinander angeordnet und ausgebildet, insbesondere gemäß einer vorbestimmten Topologie des von der Einsatz-Behälter-Aufnahme aufzunehmenden Einsatz-Behälters und;
eine Behälter-Anordnung, die offenbarungsgemäß gemäß wenigstens einem Aspekt der vorangegangenen Beschreibung ausgebildet ist, wobei der Adapter der Behälter-Anordnung in die Einsatz-Behälter-Aufnahme, vorzugsweise anstatt des vorbestimmten Einsatz-Behälters, eingesetzt ist, der Zulauf-Verbindungsabschnitt des Adapters mit dem Zulauf der Einsatz-Behälter-Aufnahme gekoppelt ist und fluidisch verbunden ist und der Ablauf-Verbindungsabschnitt des Adapters mit dem Ablauf der Einsatz-Behälter-Aufnahme zumindest gekoppelt ist, und wobei zumindest der Lösungsmittel-Strömungspfad von dem Zulauf-Verbindungsabschnitt des Adapters, über den Behälter-Einlauf des Behälters, durch den Behälter und zu dem Behälter-Auslauf vorgesehen und ausbildbar ist, insbesondere ausgebildet ist.

Die Vorteile, die diese offenbarungsgemäße extrakorporale Blutbehandlungsmaschine aufweist, wurden im Zuge der Beschreibung der offenbarungsgemäß ausgestalteten Behälter-Anordnung eingehend erläutert, sodass auf die o.g. Schilderung der Vorteile verwiesen wird, um diese Schrift nicht zu überfrachten. Kurz gesagt ist offenbarungsgemäß eine extrakorporale Blutbehandlungsmaschine zur intermittierenden oder ambulanten extrakorporalen Blutbehandlung bereitgestellt, mit der die Blutbehandlung effizienter und sicherer erfolgen kann.

Gemäß einer bevorzugten Weiterbildung hat die extrakorporale Blutbehandlungsmaschine eine Erfassungseinheit, insbesondere eine Sensoreinheit, die zumindest angepasst ist, zu erfassen, ob die Kopplungen des Zulauf-Verbindungsabschnitts des Adapters mit dem Zulauf der Einsatz-Behälter-Aufnahme und des Ablauf-Verbindungsabschnitts des Adapters mit dem Ablauf der Einsatz-Behälter-Aufnahme ausgebildet sind, oder nicht. **In** anderen Worten ausgedrückt, kann die Erfassungseinheit zumindest erfassen, ob der Adapter korrekt in die Einsatz-Behälter-Aufnahme eingesetzt ist, oder nicht. Dabei ist die Erfassungseinheit angepasst, ein von einem Ergebnis der Erfassung abhängiges Signal auszugeben, wobei eine mit der Erfassungseinheit signalverbundene Steuereinheit der Blutbehandlungsmaschine vorgesehen und angepasst ist, eine fluidische Verbindung des Lösungsmittel-Zulaufs mit dem Zulauf der Einsatz-Behälter Aufnahme nur dann aufzusteuern, wenn zumindest die beiden Kopplungen als ausgebildet erfasst sind, und zu sperren, wenn nur eine oder keine der Kopplungen als ausgebildet erfasst ist.

Die Aufsteuerung/ Sperrung der fluidischen Verbindung des Lösungsmittel-Zulaufs mit dem Zulauf der Einsatz-Behälter Aufnahme erfolgt vorzugsweise über eine Ansteuerung eines zwischen dem Lösungsmittel-Zulauf und dem Zulauf angeordneten Sperrventils.

Besonders bevorzugt ist die Erfassungseinheit ergänzend angepasst zu erfassen, ob die fluidische Verbindung zumindest des Zulauf-Verbindungsabschnitts des Adapters mit dem Zulauf der Einsatz-Behälter-Aufnahme ausgebildet ist oder nicht.

Alternativ oder ergänzend ist zumindest der Zulauf-Verbindungsabschnitt, und vorzugsweise auch der Ablauf-Verbindungsabschnitt, derart ausgestaltet, dass er bei korrekter Kopplung mit dem Zulauf der Einsatz-Behälter-Aufnahme, die dortige, fluidische Verbindung automatisch korrekt ausbildet, beispielsweise, indem der Zulauf-Verbindungsabschnitt/ bzw. der Ablauf-Verbindungsabschnitt bei Kopplung mit dem Zulauf/ bzw. mit dem Ablauf ein dortiges Rückschlagventil aufstößt oder aufsteuert und so die fluidische Verbindung automatisch mit dem Einsetzen des Adapters ausbildet.

Gemäß einer bevorzugten Weiterbildung der extrakorporalen Blutbehandlungsmaschine ist zur Bereitstellung der alkalisierenden Lösung an den Dialysierflüssigkeitskreislauf der oben genannte erste Bereitstellungs-Strömungspfad von dem Behälter-Auslauf ausgehend und unter Umgehung des Ablauf-Verbindungsabschnitts des Adapters hin zum Dialysierflüssigkeitskreislauf vorgesehen und ausbildbar, insbesondere ausgebildet. Hierzu ist vorzugsweise der Behälter-Auslauf mittels einer Leitung oder eines Schlauchs mit einer Entnahmelanze verbunden, die insbesondere in einen Lösungs-Container der extrakorporalen Blutbehandlungsmaschine, welcher zur Aufnahme und Bereitstellung der Lösung vorgesehen ist, eingesetzt oder eingetaucht ist.

Gemäß einer bevorzugten alternativen oder ergänzenden Weiterbildung der extrakorporalen Blutbehandlungsmaschine ist zur Bereitstellung der alkalisierenden Lösung an den Dialysierflüssigkeitskreislauf der oben genannte zweite Bereitstellungs-Strömungspfad von dem Behälter-Auslauf ausgehend, über den Ablauf-Verbindungsabschnitt des Adapters, den Ablauf der Einsatz-Behälter-Aufnahme und hin zum Dialysierflüssigkeitskreislauf vorgesehen und ausbildbar, insbesondere ausgebildet. Hierzu ist vorzugsweise der Behälter-Auslauf mittels einer Leitung oder eines Schlauchs mit dem Ablauf-Verbindungsabschnitt des Adapters fluidisch verbunden. Vorzugsweise ist im Falle des zweiten Bereitstellungs-Strömungspfades der Ablauf-Verbindungsabschnitt des Adapters mit dem Ablauf der Einsatz-Behälter-Aufnahme nicht nur gekoppelt, sondern auch fluidisch verbunden, sodass die alkalisierende Lösung dem Dialysierflüssigkeitskreislauf über diesen Ablauf bereitgestellt werden kann.

Vorzugsweise ist, wie bereits oben erwähnt, gemäß einer Weiterbildung der extrakorporalen Blutbehandlungsmaschine der Bypass-Strömungspfad von dem Zulauf-Verbindungsabschnitt des Adapters unter Umgehung des Behälters zu dem Ablauf-Verbindungsabschnitt des Adapters vorgesehen und ausbildbar, insbesondere ausgebildet.

Vorzugsweise ist, wie bereits oben erläutert, gemäß einer Weiterbildung der extrakorporalen Blutbehandlungsmaschine stromabwärts des Zulauf-Verbindungsabschnitts des Adapters das erste Wegeventil mit Schaltstellungen vorgesehen, wobei der Lösungsmittel-Strömungspfad in der ersten Schaltstellung des ersten Wegeventils aufgesteuert und in der zweiten Schaltstellung des ersten Wegeventils gesperrt ist.

Vorzugsweise ist, wie bereits oben erläutert, gemäß einer Weiterbildung der extrakorporalen Blutbehandlungsmaschine stromaufwärts des Ablauf-Verbindungsabschnitts des Adapters das zweite Wegeventil mit Schaltstellungen vorgesehen, wobei der zweite Bereitstellungs-Strömungspfad in der ersten Schaltstellung des zweiten Wegeventils aufgesteuert und in der zweiten Schaltstellung des zweiten Wegeventils gesperrt ist.

Vorzugsweise ist, wie bereits oben erläutert, gemäß einer Weiterbildung der extrakorporalen Blutbehandlungsmaschine der Bypass-Strömungspfad mit den ersten Schaltstellungen des ersten und zweiten Wegeventils gesperrt und mit den zweiten Schaltstellungen des ersten und zweiten Wegeventils aufgesteuert.

### Kurzbeschreibung der Figuren

Die Offenbarung wird nachfolgend anhand bevorzugter Ausführungsformen mit Hilfe von Figuren näher erläutert. Es zeigen:
- Fig. 1: einen schematischen, fluidischen Schaltplan einer extrakorporalen Blutbehandlungsmaschine gemäß einer bevorzugten Ausführungsform;
- Fig. 2: eine Behälter-Anordnung gemäß einer ersten Ausführungsform für die extrakorporale Blutbehandlungsmaschine gemäß Figur 1;
- Fig. 3: eine Behälter-Anordnung gemäß einer zweiten Ausführungsform, die in der extrakorporalen Blutbehandlungsmaschine gemäß Figur 1 verbaut ist;
- Fig. 4: eine Behälter-Anordnung gemäß einer dritten Ausführungsform für die extrakorporale Blutbehandlungsmaschine gemäß Figur 1;
- Fig. 5: die Behälter-Anordnung gemäß Figur 4 in einer Bypass-Schaltung; und
- Fig. 6 bis 8: drei Behälter der Behälter-Anordnung gemäß drei Ausführungsformen.

Die Figuren sind schematischer Natur und sollen nur dem Verständnis der Offenbarung dienen. Gleiche Elemente sind mit denselben Bezugszeichen versehen. Merkmale verschiedener Ausführungsformen können untereinander ausgetauscht werden.

### Detaillierte Beschreibung bevorzugter Ausführungsformen

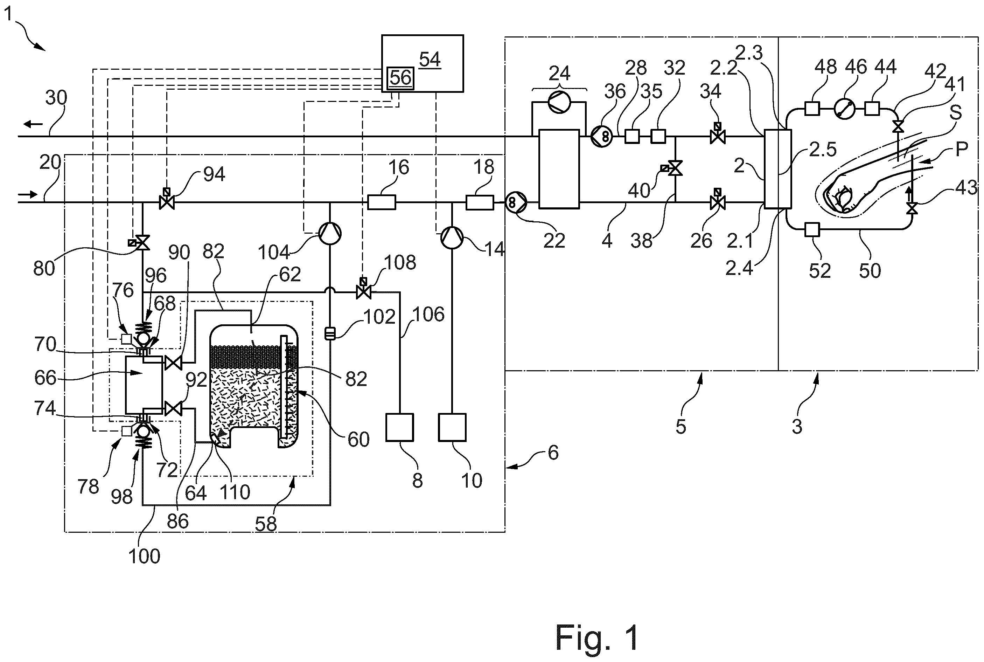

Figur 1 zeigt in einer schematischen Ansicht einen fluidischen Schaltplan einer extrakorporalen Blutbehandlungsmaschine 1 (im Folgenden nur Blutbehandlungsmaschine genannt) in Form einer Dialysemaschine für eine intermittierende, insbesondere ambulante, extrakorporale Blutbehandlung von Blut eines Patienten P gemäß einer bevorzugten Ausführungsform der vorliegenden Offenbarung.

Es werden insbesondere die Ausgestaltung und der Gebrauch einer offenbarungsgemäßen Behälter-Anordnung 58 der Blutbehandlungsmaschine 1 beschrieben, mit deren Hilfe für die intermittierende, insbesondere ambulante, extrakorporale Blutbehandlung eine alkalisierende Trockensubstanz bereitgestellt wird, in Lösung gebracht wird und die so hergestellte, alkalisierende Lösung einem Dialysierflüssigkeitskreislauf 5 der Blutbehandlungsmaschine 1 bereitgestellt wird.

Dabei erfolgt offenbarungsgemäß zumindest die Bereitstellung und das Lösen der alkalisierenden Trockensubstanz, sowie die Bereitstellung der alkalisierenden Lösung für die Blutbehandlungsmaschine 1 nicht zentralisiert, sondern an der Blutbehandlungsmaschine 1 selbst. Im Gegensatz dazu kann eine Bereitstellung und das Lösen einer sauren, salzigen oder sonstigen Trockensubstanz, sowie die Bereitstellung der entsprechenden Lösung für die Blutbehandlungsmaschine 1 durchaus zentralisiert in größeren Mischeinheiten, beispielsweise für mehrere Blutbehandlungsmaschinen 1 erfolgen, was insbesondere darin begründet ist, dass beispielsweise saure Lösungen in sich antimikrobiell sind und in großen Mengen und damit für längere Zeit vorgehalten werden können. Alkalisierende Lösungen sind demgegenüber in kleineren Mengen und zeitnah zur extrakorporalen Blutbehandlung vorzuhalten, was zur oben angesprochenen "nicht-zentralisierenden" Bereitstellung in kleinerer Menge führt.

Die Blutbehandlungsmaschine1 weist gemäß Figur 1 als zentrale Komponente einen Dialysator 2 mit einerseits je einem dialysierflüssigkeitsseitigen Dialysierflüssigkeitseingang 2.1 und Dialysatausgang 2.2 und andererseits je einem blutseitigen Bluteingang 2.3 und Blutausgang 2.4 eines extrakorporalen Blutkreislaufs 3 auf. Innerhalb ist der Dialysator 2 mittels Hohlfasern einer semipermeablen Membran 2.5 in eine Dialysierflüssigkeitsseite und eine Blutseite aufgeteilt.

Der Dialysierflüssigkeitseingang 2.1 ist über einen Dialysierflüssigkeitszulauf 4 mit einer Mischeinheit 6 fluidisch verbindbar, insbesondere verbunden. Diese stellt kontinuierlich aus zumindest teilentgastem Reinstwasser, sowie einer alkalisierenden Trockensubstanz und einem sauren Konzentrat, frische Dialysierflüssigkeit her.

Als eine Option zur Bereitstellung von einem alkalisierenden und einem sauren Konzentrat hat die Mischeinheit 6 einen ersten und zweiten Lösungs-Container 8, 10, in denen jeweils eine fertige alkalisierende und fertige saure Lösung vorgehalten sind, sowie eine erste und zweite Fördervorrichtung 12, 14 und stromabwärts der Fördervorrichtungen 12, 14 jeweils eine erste und zweite Messvorrichtung 16, 18.

Gemäß Figur 1 hat die Blutbehandlungsmaschine 1 einen Lösungsmittel-/ Reinstwasser-Zulauf 20, an dem fortlaufend von einer internen Entgasungseinheit (nicht dargestellt) zumindest teilentgastes Reinstwasser bereitgestellt wird. Der Reinstwasser-Zulauf 20 ist über ein Sperrventil 94, das mit einer Steuereinheit 54 der Blutbehandlungsmaschine 1 signalverbunden ist, fluidisch mit den stromabwärts in Reihe angeordneten Messvorrichtungen 16, 18, einer Fördereinrichtung 22 und einer Bilanzierungsvorrichtung 24 verbindbar, insbesondere verbunden. Ausgangsseitig ist die Bilanzierungsvorrichtung 24 über einen Dialysierflüssigkeitszulauf 4 fluidisch mit dem Dialysierflüssigkeitseingang 2.1 des Dialysators 2 verbindbar, insbesondere verbunden, wobei im Dialysierflüssigkeitszulauf 4 ein Ventil 26 zum Absperren des Dialysierflüssigkeitseingangs 2.1 angeordnet ist.

Der Dialysatausgang 2.2 ist über einen Dialysatablauf 28 mit einem Entsorgungsausgang 30 für verbrauchte Dialysierflüssigkeit/ Dialysat fluidisch verbindbar, insbesondere verbunden. Im Dialysatablauf 28 sind, zwischen dem Dialysatausgang 2.2 und dem Entsorgungsausgang 30, fluidisch in Reihe angeordnet: ein betätigbares Ventil 34 zum Absperren des Dialysatausgangs 2.2, eine Erfassungseinheit 32 zur Erfassung eines Bestandteils im Dialysat und eine vierte Fördervorrichtung 36, über die das Dialysat zur Bilanzierungsvorrichtung 24 und zum Entsorgungsausgang 30 für Dialysat gefördert wird. Die Bilanzierungsvorrichtung 24 sorgt dafür, dass mittels einer Ultrafiltrationspumpe ein gewünschtes Volumen an überschüssigem Wasser im Rahmen einer Ultrafiltration aus dem Patientenblut entzogen werden kann. Stromaufwärts der vierten Fördervorrichtung 36 ist im Dialysatablauf 28 eine Druckerfassungseinheit 35 zur Erfassung eines Dialysatausgangs-Drucks vorgesehen.

Ergänzend ist ein Bypass-Strömungspfad 38 vorgesehen, über den der Dialysierflüssigkeitszulauf 4 mit dem Dialysatablauf 28 fluidisch verbindbar ist. In dem Bypass-Strömungspfad 38 ist ein betätigbares Ventil 40 angeordnet, über das der Bypass-Strömungspfad 38 sperrbar ist.

Auf der Seite des Blutes ist der extrakorporale Blutkreislauf 3 vorgesehen, welcher über einen arteriellen Schlauchabschnitt 42 dem Patienten P Blut entnehmen und über den Bluteingang 2.3 dem Dialysator 2 zuführen kann. In dem arteriellen Schlauchabschnitt 42 sind in Strömungsrichtung eine arterielle Schlauchklemme 41, ein arterieller Hämatokrit-Sensor oder HCT-Sensor 44, eine Blutpumpe 46 und ein Bluteingangs-Druck-Sensor 48 angeordnet. Nachdem im extrakorporalen Blutkreislauf 3 das Blut des Patienten P durch die Blutseite des Dialysators 2 geleitet wurde, wird es an dessen Blutausgang 2.4 entnommen und über einen venösen Schlauchabschnitt 50 dem Shunt S zugeführt. In dem venösen Schlauchabschnitt 50 sind ein Blutausgangs-Druck-Sensor 52 und eine venöse Schlauchklemme 43 angeordnet. In dem Dialysator 2 wird das Blut im Gegenstromverfahren zur Dialysierflüssigkeit geführt und von harnpflichtigen Bestandteilen und überschüssigem Wasser befreit und hiernach gereinigt dem Patienten P zurückgegeben/ zurückgeführt.

Gemäß Figur 1 hat die extrakorporale Blutbehandlungsmaschine 1 offenbarungsgemäß eine Behälter-Anordnung 58 zur Herstellung und Bereitstellung einer alkalisierenden Lösung an den Dialysierflüssigkeitskreislauf 5. Die offenbarungsgemäße Behälter-Anordnung 58 kann dabei ergänzend zu dem oder anstatt des Containers 8 mit bereits fertiger/ vorgemischter alkalisierender Lösung bereitgestellt werden.

Von dem Reinstwasser-Zulauf 20 zweigt stromaufwärts des Sperrventils 94 ein Zulaufpfad ab, in dem ein mit der Steuereinheit signalverbundenes und betätigbares Sperrventil 80 und stromabwärts davon ein in Strömungsrichtung schließendes und federbelastetes Rückschlagventil 96 angeordnet sind. Der Zulaufpfad endet in einer Aufnahme 68 einer Einsatz-Behälter-Aufnahme 68, 72 der Mischeinheit 6. Diametral gegenüber der Aufnahme 68 hat die Einsatz-Behälter-Aufnahme 68, 72 einen Ablauf 72. An diesem ist in spiegelbildlicher Anordnung und dementsprechend entgegengesetzter Schließrichtung und Federbelastung ein Rückschlagventil 98 vorgesehen.

Am Ablauf 72 der Einsatz-Behälter-Aufnahme 68, 72 setzt ein Bereitstellungspfad 100 an, der über einen Filter 102 und eine mit der Steuereinheit 54 signalverbundene und steuerbare Fördervorrichtung 104 stromabwärts des Sperrventils 94 und stromaufwärts der ersten Messvorrichtung 16 in den vom Reinstwasser-Zulauf 20 kommenden Strömungspfad einmündet.

Von dem Container 8, von dem bereits fertige/ vorgemischte alkalisierende Lösung bereitgestellt werden kann, geht ein Bereitstellungspfad 106 aus, der über ein mit der Steuereinheit 54 signalverbundenes und betätigbares Sperrventil 108 verläuft und in den Zulaufpfad zwischen dem Sperrventil 80 und dem Rückschlagventil 96 mündet.

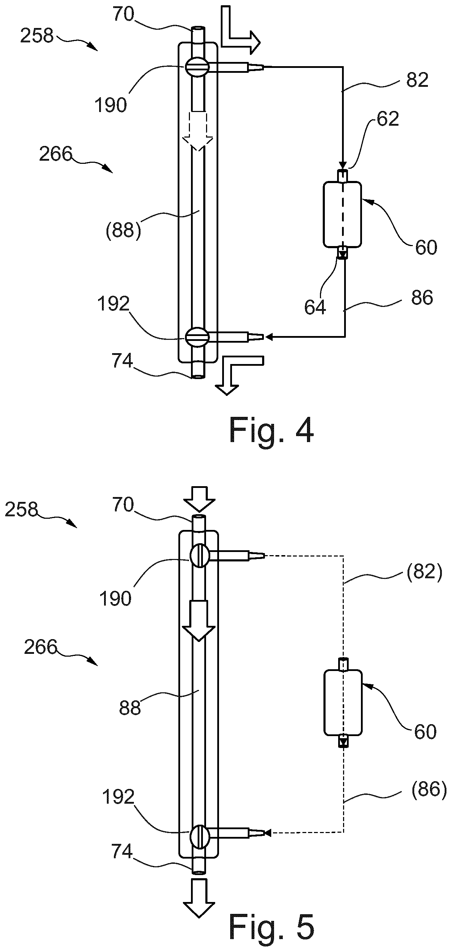

Die offenbarungsgemäße Behälter-Anordnung 58 gemäß Figur 1 hat als erste Komponente einen Adapter 66 mit einem Zulauf-Verbindungsabschnitt 70 und einem Ablauf-Verbindungsabschnitt 74. Als zweite Komponente hat sie einen die alkalisierende Trockensubstanz beinhaltenden Behälter 60 mit einem Behälter-Einlauf 62 und einem Behälter-Auslauf 64. In dem Behälter 60 steht gemäß der Darstellung in Figur 4 bereits das Lösungsmittel, welches (wie im nachfolgenden noch beschrieben wird) über den Zulauf 68 der Einsatz-Behälter-Aufnahme 68, 72 zugeführt wird/ wurde. Zudem hat die offenbarungsgemäße Behälter-Anordnung 58 gemäß Figur 1 einen Lösungsmittel-Strömungspfad 82, über den der Zulauf-Verbindungsabschnitt 70 des Adapters 66 mit dem Behälter-Einlauf 62 fluidisch verbunden ist. In dem Lösungsmittel-Strömungspfad 82 ist ein manuell betätigbares, als 2/2-Wegeventil ausgestaltetes, erstes Wegeventil 90 angeordnet, über das der Behälter-Einlauf 62 mit dem Zulauf-Verbindungsabschnitt 70 des Adapters 66, je nach Schaltstellung, fluidisch verbunden oder getrennt werden kann.

Der Lösungsmittel-Strömungspfad 82 erstreckt sich über den Behälter-Einlauf 62 hinaus, durch den Behälter 60 und die darin befindliche Trockensubstanz/ Lösung hindurch, zum Behälter-Auslauf 64, an dem ein Filter 110 zur Zurückhaltung ungelöster Trockensubstanz vorgeschaltet ist.

Über einen zweiten Bereitstellungspfad 86 ist der Behälter-Auslauf 64 fluidisch mit dem Ablauf-Verbindungsabschnitt 74 des Adapters 66 verbindbar, insbesondere verbunden, wobei im zweiten Bereitstellungspfad 86 - insbesondere in Analogie zum ersten Abschnitt des Lösungsmittelströmungspfades 82 vom Zulauf-Verbindungsabschnitt 70 zum Behälter-Einlauf - ein manuell betätigbares, als 2/2-Wegeventil ausgestaltetes, zweites Wegeventil 90 angeordnet ist, über das der Behälter-Auslauf 64 mit dem Ablauf-Verbindungsabschnitt 74 des Adapters 66, je nach Schaltstellung, fluidisch verbunden oder getrennt werden kann.

Gemäß Figur 1 ist die Behälter-Anordnung aus Adapter 66, Behälter 60, Lösungsmittel-Strömungspfad 82, zweitem Bereitstellungspfad 86, sowie den beiden Wegeventilen 90, 92 bestimmungsgemäß an die Einsatz-Behälter-Aufnahme 68, 72 angeschlossen. Das heißt, dass die Verbindungsabschnitte 70, 74 des Adapters 66 korrekt mit der ihr jeweils zugeordnete Aufnahme 68, 72 der Einsatz-Behälter-Aufnahme 68, 72 gekoppelt sind. Ob die Kopplung korrekt ausgebildet ist, wird von einer mit der Steuereinheit 54 signalverbundenen Erfassungseinheit 76, 78 erfasst und an die Steuereinheit 54 gemeldet. Insbesondere wird gemeldet, ob tatsächlich beide Verbindungsabschnitte 70, 74 mit der jeweiligen Aufnahme 68, 72 korrekt gekoppelt sind, oder nur eine oder keine. Die Erfassungseinheit 76, 78 kann beispielsweise an jeder einzelnen der Aufnahmen 68, 72 von einer Lichtschranke oder einem Kontaktschalter gebildet sein, die/ der bei korrekt gekoppeltem Verbindungsabschnitt 70, 74 ein entsprechendes Signal an die Steuereinheit 54 übermittelt.

Die Einsatz-Behälter-Aufnahme 68, 72 der offenbarungsgemäßen Blutbehandlungsmaschine 1 weist eine vorbestimmte Anschluss-Topologie, das heißt, eine vorbestimmte Anordnung der Zulauf-Aufnahme 68 relativ zu der Ablauf-Aufnahme 72, sowie vorzugsweise vorbestimmte Abmessungen der Zulauf-Aufnahme 68 und der Ablauf-Aufnahme 72 auf.

Auf diese Weise ist sie vorbereitet, einen spezifischen/ vorbestimmten Einsatz-Behälter (nicht dargestellt) der alkalisierenden Trockensubstanz, der dieser vorbestimmten Anschluss-Topologie als Gegenstück entspricht, aufzunehmen, jeweils mit dem Zulauf 68 und dem Ablauf 72 zu koppeln und fluidisch zu verbinden. Die vorbestimmte Anschluss-Topologie der Einsatz-Behälter-Aufnahme 58 hat den Vorteil, dass in die Einsatz-Behälter-Aufnahme 58 als Gegenstück kein Behälter aufgenommen, gekoppelt und fluidisch verbunden werden kann, der die vorbestimmte Anschluss-Topologie der Einsatz-Behälter-Aufnahme 58 nicht erfüllt oder ihr nicht entspricht.

Umgekehrt kann natürlich der spezifische/ vorbestimmte Einsatz-Behälter, das heißt der Einsatz-Behälter mit seiner vorbestimmten Anschluss-Topologie nur in diese und keine andere Einsatz-Behälter-Aufnahme mit abweichender Anschluss-Topologie eingesetzt, gekoppelt und fluidisch verbunden werden.

Beides bringt mit sich, dass Verwechslungen ausgeschlossen sind.

Das bringt allerdings mit sich, dass der verfügbare, spezifische vorbestimmte Einsatz-Behälter der alkalisierenden Trockensubstanz eine vergleichsweise geringe Größe aufweist und dementsprechend wenig Trockensubstanz beinhaltet. Die Menge ist dabei auf eine einmalige Blutbehandlung zur Abdeckung auch langer Blutbehandlungen hin optimiert und somit mit einer Menge bestückt, die zwar relativ klein ist aber dennoch selten voll ausgeschöpft wird. Der Einsatz-Behälter ist entsprechend zur Einmalverwendung, bzw. als "single use"-Produkt konzipiert. Damit verbunden sind ein häufiges Wechseln, eine bei nahezu jedem Wechsel anfallende Verwurfsmenge, sowie eine erhebliche Menge an Kunststoffabfall aufgrund des anfallenden Verpackungsmaterials.

Um diese Nachteile zu beheben oder zu mindern stellt die offenbarungsgemäße Behälter-Anordnung den Adapter 66 bereit, der eine an die Anschluss-Topologie der Einsatz-Behälter-Aufnahme 68, 72 angepasste Topologie, insbesondere des Zulauf-Verbindungsabschnitts 70 und des Ablauf-Verbindungsabschnitt 72 hat.

Dementsprechend passt bei in die Einsatz-Behälter-Aufnahme 68, 72 eingesetztem Adapter 66 der Zulauf-Verbindungsabschnitt 70 genau in den Zulauf 68 und stößt dort das Rückschlagventil 96 auf und der Ablauf-Verbindungsabschnitt 74 passt genau in den Ablauf 72 und stößt dort das Rückschlagventil 98 auf. Entsprechend ist der Zulauf-Verbindungsabschnitt 70 mit dem Zulauf 68 und der Ablauf-Verbindungsabschnitt 74 mit dem Ablauf 72 jeweils gekoppelt und fluidisch verbunden.

Der Steuereinheit 54 wird dieser Zustand von der Erfassungseinheit 76, 78 gemeldet, sodass von ihr in Folge das Sperrventil 80 aufgesteuert und das Sperrventil 94 zugesteuert werden kann. Auf diese Weise steht am ersten Wegeventil der Behälter-Anordnung 58 Reinstwasser an. Als nächstes können von einem Bedienpersonal die beiden Wegeventile 90, 92 von ihrer sperrenden, zweiten Schaltstellung in ihre offene, erste Schaltstellung betätigt werden. Eine weitere Randbedingung ist, dass das Sperrventil 108 zugesteuert ist.

So strömt Reinstwasser vom Reinstwasser-Zulauf 20, über das Sperrventil 80, das aufgestoßene Rückschlagventil 96, den Zulauf 68, den Zulauf-Verbindungsabschnitt 70 des Adapter 66, das aufgesteuerte erste Wegeventil 90, den Behälter-Einlauf 62 und entlang dem Lösungsmittel-Strömungspfad 82 durch den Behälter 60 und löst das darin beinhaltete Natrium-Bicarbonat. Über den Filter 110 und den Behälter-Auslauf 64 tritt die Bicarbonat-Lösung aus dem Behälter 60 aus und strömt über den zweiten Bereitstellungs-Strömungspfad 86, das aufgesteuerte zweite Wegeventil 92, den Ablauf-Verbindungsabschnitt 74 des Adapters 66, das aufgestoßene Rückschlagventil 98 und den Bereitstellungspfad 100 dem Dialysier-Flüssigkeits-Kreislauf 5 zu.

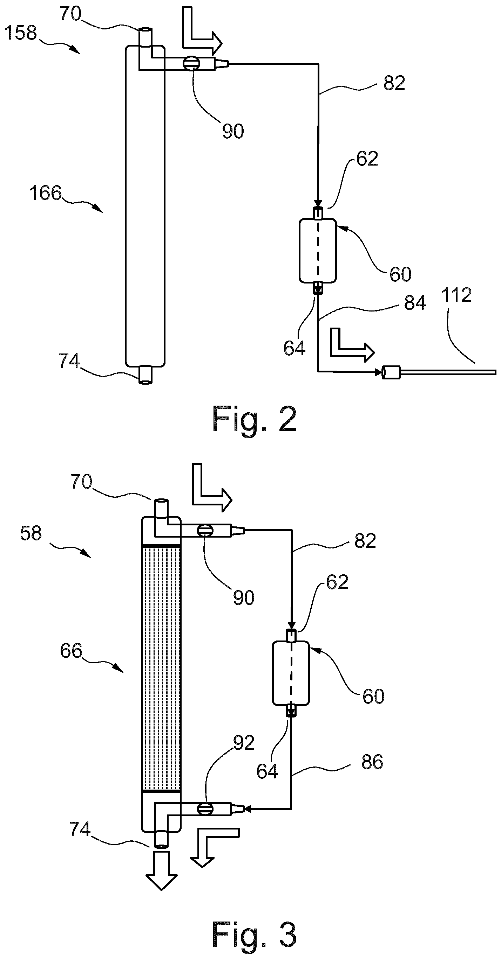

Die folgenden Figuren 2 bis 5 zeigen Ausführungsformen einer offenbarungsgemäßen Behälter-Anordnung 58; 158; 258, wobei die Ausführungsform gemäß der Figur 3 der in Figur 1 gezeigten Ausführungsform der Behälter-Anordnung 58 entspricht.

Alle Ausführungsformen der in den Figuren 2 bis 5 gezeigten Behälter-Anordnungen 58; 158; 258 sind geeignet, mit der Einsatz-Behälter-Aufnahme 68, 72 der Blutbehandlungsmaschine 1 gemäß Figur 1 gekoppelt und fluidisch verbunden zu werden, da ihr jeweiliger Adapter 66; 166; 266 topologisch, insbesondere bezüglich seiner Anschluss-Topologie, so ausgestaltet ist, dass er mit seinem Zulauf- und Ablauf-Verbindungsabschnitt 70, 74 in die Einsatz-Behälter-Aufnahme 68, 72 eingesetzt und mit dem Zulauf 68 und Ablauf 72 gekoppelt werden kann (vgl. Figur 1). Je nach Ausführungsform wird (werden) dann die entsprechende(n) fluidische(n) Verbindung(en) ausgebildet.

Vorab sei erwähnt, dass der Behälter 60 in den Figuren 2 bis 5 schematisch dargestellt ist und im Wesentlichen nur dazu dargestellt wird, um die unterschiedlichen Bereitstellungs-Strömungspfade der alkalisierenden Lösung zu verdeutlichen.

Figur 2 zeigt eine Behälter-Anordnung 158 in einer Ausführungsform, die vergleichsweise einfach ausgestaltet ist. Der Adapter 166 ist so ausgestaltet, dass zwar (wie oben erwähnt) der Zulauf-Verbindungsabschnitt 70 und der Ablauf-Verbindungsabschnitt 74 in ihre jeweilige Einsatz-Behälter-Aufnahme 68, 72 (vgl. Figur 1) eingesetzt und mit dem ihnen zugeordneten Zulauf 68 bzw. Ablauf 72 gekoppelt werden können. Allerdings ist nur der Zulauf-Verbindungsabschnitt 70 zur fluidischen Verbindung mit dem Zulauf 68 vorgesehen. Der Ablauf-Verbindungsabschnitt 74 bleibt fluidisch inaktiv oder "blind" und erfüllt lediglich die vorbeschriebene Funktion, den Ablauf 72 zu belegen oder zu besetzen, sodass die Erfassungseinheit 76, 78 (vgl. Figur 1) an die Steuereinheit 54 meldet, dass die Einsatz-Behälter-Aufnahme 68, 72 korrekt gekoppelt ist. Dementsprechend erstreckt sich der Lösungsmittel-Strömungspfad 82 vom Zulauf-Verbindungsabschnitt 70, über das erste Wegeventil 90, das als manuell betätigbares 2/2-Wegeventil mit zwei Schaltstellungen ausgestaltet ist, zum Behälter-Einlauf und durch den Behälter 60 hindurch. Vom Behälter-Auslauf 64 erstreckt sich ein erster Bereitstellungs-Strömungspfad 84, an den eine Lanze 112 angeschlossen ist. Diese Lanze 112 kann beispielsweise in den Container 8 eingesetzt werden, um diesen zu befüllen. Das erste Wegeventil 90 ist in seine erste Schaltstellung geschaltet, sodass der Lösungsmittel-Strömungspfad 82 und der erste Bereitstellungs-Strömungspfad 84 ausgebildet sind. In seiner zweiten Schaltstellung (nicht dargestellt) ist der Lösungsmittel-Strömungspfad 82 hingegen gesperrt und dem Behälter 60 läuft kein Reinstwasser mehr zu.

Figur 3 zeigt die Ausführungsform der Behälter-Anordnung 58, die in der extrakorporalen Blutbehandlungsmaschine gemäß Figur 1 verbaut ist. Die Wegeventile 90, 92 sind in ihre erste Schaltstellung geschaltet, sodass der Lösungsmittel-Strömungspfad 82 und der zweite Bereitstellungs-Strömungspfad 86 ausgebildet sind. In ihrer zweiten Schaltstellung (nicht dargestellt) ist der Lösungsmittel-Strömungspfad 82 hingegen gesperrt, dem Behälter 60 läuft kein Reinstwasser mehr zu und dem Ablauf-Verbindungsabschnitt 74 läuft keine alkalisierende Lösung mehr zu.

Die Figuren 4 und 5 zeigen eine Behälter-Anordnung 258 gemäß einer dritten Ausführungsform. Vom Zulauf-Verbindungsabschnitt 70 zweigt hierbei innerhalb des Adapters 266 ein Bypass-Strömungspfad 88 ab, über den der Zulauf-Verbindungsabschnitt 70 unter Umgehung des Behälters 60 direkt mit dem Ablauf-Verbindungsabschnitt 74 des Adapters 266 verbunden werden kann. Um den Lösungsmittel-Strömungspfad 82 über den Behälter 60 und den Bypass-Strömungspfad 88 schalten zu können, sind die Wegeventile 190, 192 als manuell betätigbare 3/2-Wegeventile mit drei Anschlüssen und zwei Schaltstellungen ausgebildet, sodass in den ersten Schaltstellungen der Wegeventile 190, 192 (vgl. Figur 4) der Bypass-Strömungspfad gesperrt und der Lösungsmittel-Strömungspfad 82 und der zweite Bereitstellungs-Strömungspfad 86 aufgesteuert sind, wohingegen in den zweiten Schaltstellungen (vgl. Figur 5) der Wegeventile 190, 192 der Bypass-Strömungspfad aufgesteuert und der Lösungsmittel-Strömungspfad 82 und der zweite Bereitstellungs-Strömungspfad 86 gesperrt sind. Mit Hilfe des Bypass-Strömungspfades im Adapter 266 kann vorzugsweise eine Desinfektion des fluidischen Systems vom Lösungsmittel-/ Reinstwasser-Zulauf 20 über die Einsatz-Behälter-Aufnahme 68, 72 und den Dialysierflüssigkeitskreislauf 5 durch Spülung mit einer Desinfektionsflüssigkeit desinfiziert werden, ohne dass der Behälter 60 gewechselt werden muss. Dieser wird während der Desinfektion einfach fluidisch umgangen (vgl. Figur 5) und nach der Desinfektion durch einfaches Umschalten der Wegeventile 190, 192 in die ersten Schaltstellungen (vgl. Figur 4) wieder "aktiviert".

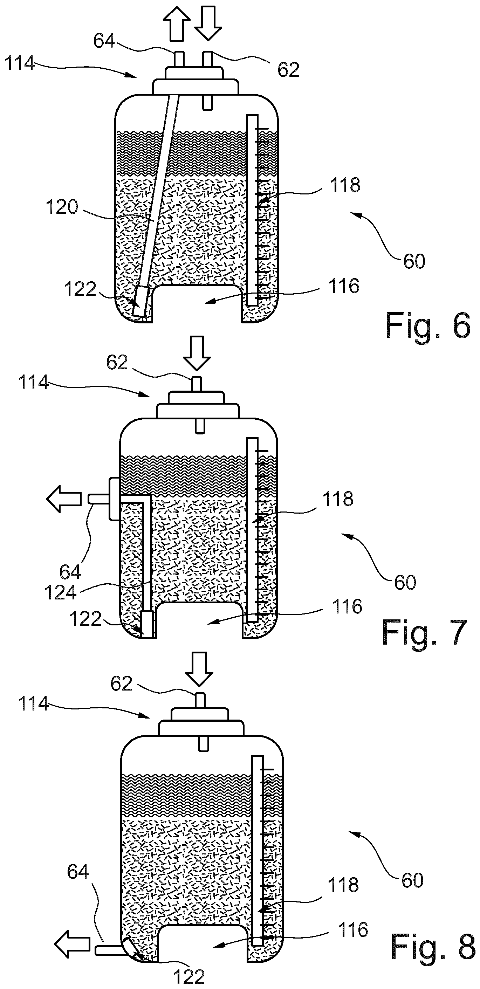

Die Figuren 6 bis 8 zeigen drei Ausführungsformen des Behälters 60, die sich darin unterscheiden, wo der Behälter-Auslauf angeordnet ist und wie der Lösungsmittel-Strömungspfad innerhalb des Behälters verläuft.

Gemeinsam ist den Ausführungsformen gemäß den Figuren 6 bis 8, dass der Behälter 60 eine zylindrische oder quaderförmige Grundform mit abgerundeten Kanten und/ oder Ecken hat. An einer Oberseite ist ein Schraubdeckel 114 angeordnet, der in allen Fällen von dem als Einlauf-Rohrstück ausgebildeten Behälter-Einlauf 62 durchgriffen ist. Durch das vergleichsweise kurze Einlauf-Rohrstück 62 tritt so das Lösungsmittel/ Reinstwasser in den Behälter 60 ein. Diametral zum Schraubdeckel 114 weist der Behälter bodenseitig eine Aussparung 116 auf, die etwas größer dimensioniert ist als der Schraubdeckel 114. So ist es möglich, mehrere solcher Behälter 60 zu stapeln, wobei der Schraubdeckel 114 eines unteren Behälters 60 in die Aussparung 116 des jeweils darüber angeordneten Behälters 60 eingreift, wodurch die gestapelten Behälter 60 lagefixiert/ positioniert werden. Der Behälter 60 weist zudem eine Skala 118 auf und ist aus transparentem Kunststoff, beispielsweise PE oder PET gefertigt, sodass stets sichtbar ist, wieviel Trockensubstanz/ angelöste Trockensubstanz noch im Behälter 60 enthalten ist.

Ein weiterer Effekt der o.g. Aussparung 116 ist, dass so außenumfänglich zur Aussparung 116 ein ringförmiger, vergleichsweise schmaler Bereich im Behälter 60 ausgebildet ist, der sich eignet, dass hier eine Entnahme der alkalisierenden Lösung erfolgt. In allen drei Ausführungsformen des Behälters 60 gemäß den Figuren 6 bis 8 erfolgt diese Entnahme an diesem tiefsten Punkt oder Bereich.

Der Unterschied der Ausführungsformen des Behälters 60 gemäß den Figuren 6 bis 8 liegt allein in der Anordnung des Behälter-Auslaufs 64.

Gemäß Figur 6 ist der Behälter-Auslauf 64 am Schraubdeckel 114 ausgebildet, wobei ein Tauchrohr 120 vorgesehen ist, das sich vom tiefsten Punkt/ Bereich zum Schraubdeckel 114 erstreckt und diesen durchgreift. Am tiefsten Punkt ist eine Mündung des Tauchrohrs 120 von einem Filter 122 abgedeckt, um das Eindringen noch ungelöster Trockensubstanz in das Tauchrohr 120 zu verhindern.

Gemäß Figur 7 ist der Behälter-Auslauf 64 randseitig am Behälter 60 ausgebildet, wobei ein Tauchrohr 124 vorgesehen ist, das sich vom tiefsten Punkt/ Bereich zum randseitigen Behälter-Auslauf 64 erstreckt. Auch hier ist am tiefsten Punkt eine Mündung des Tauchrohrs 122 von dem Filter 122 abgedeckt, um das Eindringen noch ungelöster Trockensubstanz in das Tauchrohr 120 zu verhindern.

Gemäß Figur 8 ist der Behälter-Auslauf 64 randseitig am tiefsten Punkt/ Bereich des Behälters 60 ausgebildet, sodass auf ein Tauchrohr verzichtet werden kann. In diesem Fall ist einer Mündung des Behälter-Auslaufs 64 in den Behälter der Filter 122 vorgeschaltet, um das Eindringen noch ungelöster Trockensubstanz in den Behälter-Auslauf 64 zu verhindern.

### Bezugszeichenliste

- 1: extrakorporale Blutbehandlungsmaschine
- 2: Dialysator
- 2.1: Dialysierflüssigkeitseingang
- 2.2: Dialysatausgang
- 2.3: Bluteingang
- 2.4: Blutausgang
- 2.5: semipermeable Membran
- 3: extrakorporaler Blutkreislauf
- 4: Dialysierflüssigkeitszulauf
- 5: Dialysierflüssigkeitskreislauf
- 6: Mischeinheit
- 8: Container alkalisierende Lösung
- 10: Container saure Lösung
- 12: erste Fördervorrichtung
- 14: zweite Fördervorrichtung
- 16: erste Messvorrichtung
- 18: zweite Messvorrichtung
- 20: Lösungsmittel-Zulauf/ Reinstwasser-Zulauf
- 22: Fördervorrichtung
- 24: Bilanziervorrichtung
- 26: erstes Ventil
- 28: Dialysatablauf
- 30: Entsorgungsausgang
- 32: Erfassungseinheit
- 34: zweites Ventil
- 38: Bypass-Strömungspfad (Dialysierflüssigkeitskreislauf)
- 40: drittes Ventil
- 41: arterielle Schlauchklemme
- 42: arterieller Schlauchabschnitt
- 43: venöse Schlauchklemme
- 44: Blutbestandteil-Sensor
- 46: Blutpumpe
- 48: Bluteingangs-Druck-Sensor
- 50: venöser Schlauchabschnitt
- 52: Blutausgangs-Druck-Sensor
- 54: Steuereinheit
- 56: Speicher
- 58; 158; 258: Behälter-Anordnung
- 60: Behälter alkalisierende Trockensubstanz
- 62: Behälter-Einlauf
- 64: Behälter-Auslauf
- 66; 166; 266: Adapter
- 68: Zulauf
- 70: Zulauf-Verbindungsabschnitt
- 72: Ablauf
- 74: Ablauf-Verbindungsabschnitt
- 76, 78: Erfassungseinheit
- 80: Sperrventil
- 82: Lösungsmittel-Strömungspfad
- 84: erster Bereitstellungs-Strömungspfad
- 86: zweiter Bereitstellungs-Strömungspfad
- 88: Bypass-Strömungspfad
- 90; 190: erstes Wegeventil
- 92; 192: zweites Wegeventil
- 94: Sperrventil
- 96: Rückschlagventil
- 98: Rückschlagventil
- 100: Bereitstellungspfad
- 102: Filter
- 104: Fördervorrichtung
- 106: Bereitstellungspfad
- 108: Sperrventil
- 110: Filter
- 112: Lanze
- 114: Schraubdeckel
- 116: Aussparung
- 118: Skala
- 120: Tauchrohr
- 122: Filter
- 124: Tauchrohr

- P: Patient
- S: Shunt

## Patentansprüche

1. Behälter-Anordnung (58) für eine oder einer extrakorporalen Blutbehandlungsmaschine (1), die zur intermittierenden oder ambulanten Blutbehandlung vorgesehen und ausgestaltet ist, wobei die Behälter-Anordnung als Ersatz eines mit vorbestimmter Topologie ausgestalteten Einsatz-Behälters der extrakorporalen Blutbehandlungsmaschine (1), zur Bereitstellung einer alkalisierenden Lösung, insbesondere einer Bicarbonat-Lösung, vorgesehen und ausgestaltet ist, aufweisend:
- einen Behälter (60), der vorgesehen und ausgebildet ist, die alkalisierende Trockensubstanz aufzunehmen, mit einem Lösungsmittel in Kontakt zu bringen und die alkalisierende Lösung bereitzustellen, mit einem Behälter-Einlauf (62), der zur fluidischen Verbindung mit einem Lösungsmittel-Zulauf (20) der Blutbehandlungsmaschine (1) vorgesehen und ausgebildet ist, und einem Behälter-Auslauf (64), der zur Bereitstellung der alkalisierenden Lösung, insbesondere an einen Dialysierflüssigkeitskreislauf (5) der extrakorporale Blutbehandlungsmaschine (1), vorgesehen und ausgebildet ist,
- einen Adapter (66), der zum Einsetzen in eine Einsatz-Behälter-Aufnahme (68, 72) der extrakorporalen Blutbehandlungsmaschine (1) vorgesehen und ausgestaltet ist, mit einem Zulauf-Verbindungsabschnitt (70), der zur Kopplung und fluidischen Verbindung mit einem Zulauf (68) der Einsatz-Behälter-Aufnahme (68, 72) vorgesehen und ausgestaltet ist, und mit einem Ablauf-Verbindungsabschnitt (74), der zumindest zur Kopplung, vorzugsweise auch zur fluidischen Verbindung, mit einem Ablauf (72) der Einsatz-Behälter-Aufnahme (68, 72) vorgesehen und ausgestaltet ist, und
- einen Lösungsmittel-Strömungspfad (82), der von dem Zulauf-Verbindungsabschnitt (70) des Adapters (66), über den Behälter-Einlauf (62), durch den Behälter (60) und zu dem Behälter-Auslauf (64) vorgesehen und ausbildbar ist.

2. Behälter-Anordnung (158) nach Anspruch 1, **dadurch gekennzeichnet, dass** zur Bereitstellung an den Dialysierflüssigkeitskreislauf (5) der extrakorporalen Blutbehandlungsmaschine (1) ein Bereitstellungs-Strömungspfad (84) von dem Behälter-Auslauf (64) ausgehend und unter Umgehung des Ablauf-Verbindungsabschnittes (74) des Adapters (166) vorgesehen ist.

3. Behälter-Anordnung (58; 258) nach Anspruch 1, **dadurch gekennzeichnet, dass** zur Bereitstellung der alkalisierenden Lösung an den Dialysierflüssigkeitskreislauf (5) der extrakorporalen Blutbehandlungsmaschine (1) ein Bereitstellungs-Strömungspfad (86) von dem Behälter-Auslauf (64) ausgehend und zu dem Ablauf-Verbindungsabschnitt (74) des Adapters (66; 266) vorgesehen ist.

4. Behälter-Anordnung (58) nach Anspruch 3, **dadurch gekennzeichnet, dass** unter Umgehung des Behälters (60) ein Bypass-Strömungspfad (88) von dem Zulauf-Verbindungsabschnitt (70) des Adapters (266) zu dem Ablauf-Verbindungsabschnitt (74) des Adapters (266) vorgesehen ist.

5. Behälter-Anordnung (58; 158; 258) nach Anspruch 3 oder 4, **dadurch gekennzeichnet, dass** stromabwärts des Zulauf-Verbindungsabschnitt (70) des Adapters (66; 166; 266) ein erstes Wegeventil (90; 190) mit Schaltstellungen vorgesehen ist, wobei der Lösungsmittel-Strömungspfad (82) in einer ersten Schaltstellung des ersten Wegeventils (90; 190) aufgesteuert und in einer zweiten Schaltstellung des ersten Wegeventils (90; 190) gesperrt ist.

6. Behälter-Anordnung (58; 258) einem der Ansprüche 3 bis 5, **dadurch gekennzeichnet, dass** stromaufwärts des Ablauf- Verbindungsabschnitt (74) des Adapters (66; 266) ein zweites Wegeventil (92; 192) mit Schaltstellungen vorgesehen ist, wobei der Bereitstellungs-Strömungspfad (86) in einer ersten Schaltstellung des zweiten Wegeventils (92; 192) aufgesteuert und in einer zweiten Schaltstellung des zweiten Wegeventils (92; 192) gesperrt ist.

7. Behälter-Anordnung (58) nach den Ansprüchen 4 bis 6, **dadurch gekennzeichnet, dass** der Bypass-Strömungspfad (88) mit den ersten Schaltstellungen der beiden Wegeventile (190, 192) gesperrt und mit den zweiten Schaltstellungen der beiden Wegeventile (190, 192) aufgesteuert ist.

8. Behälter-Anordnung (58; 158; 258) nach einem der vorhergehenden Ansprüche, **dadurch gekennzeichnet, dass** der Behälter-Einlauf (62) und der Behälter-Auslauf (64) am höchsten Punkt oder Bereich des Behälters (60) vorgesehen sind, wobei sich vom Behälter-Auslauf (64) ein Tauchrohr (120) in den Behälter (60) erstreckt, welches im tiefsten Punkt oder Bereich des Behälters (60) mündet, oder dass der Behälter-Einlauf (62) am höchsten Punkt oder Bereich des Behälters (60) vorgesehen ist und der Behälter-Auslauf (64) randseitig vorgesehen ist, wobei sich vom Behälter-Auslauf (64) ein Tauchrohr (124) in den Behälter (60) erstreckt, welches im tiefsten Punkt oder Bereich des Behälters (60) mündet, oder dass der Behälter-Einlauf (62) am höchsten Punkt oder Bereich des Behälters (60) vorgesehen ist und der Behälter-Auslauf (64) am tiefsten Punkt oder Bereich des Behälters (60) vorgesehen ist.

9. Extrakorporale Blutbehandlungsmaschine (1), insbesondere Dialysemaschine, die zur intermittierenden oder ambulanten extrakorporalen Blutbehandlung von Blut eines Patienten (P) vorgesehen und ausgebildet ist, zumindest aufweisend:
- einen Dialysator (2),
- einen Dialysierflüssigkeitskreislauf (5), welcher über einen Dialysierflüssigkeitseingang (2.1) und einen Dialysatausgang (2.2) des Dialysators (2) durch den Dialysator (2) verläuft,
- eine Mischeinheit (6), die dafür vorgesehen und angepasst ist, zumindest Reinstwasser und eine alkalisierende Trockensubstanz, vorzugsweise eine Bicarbonat-Trockensubstanz, zu einer alkalisierenden Lösung zu mischen und dem Dialysierflüssigkeitskreislauf (5) bereitzustellen,
- eine Einsatz-Behälter-Aufnahme (68, 72), insbesondere der Mischeinheit (6), die zur Kopplung und fluidischen Verbindung mit einem mit vorbestimmter Topologie ausgestalteten Einsatz-Behälter zur Bereitstellung der alkalisierenden Lösung vorgesehen und ausgestaltet ist, und die einen Zulauf (68) hat, der mit einem Lösungsmittel-Zulauf (20) der extrakorporalen Blutbehandlungsmaschine (1) fluidisch verbindbar, insbesondere verbunden, ist, und die einen Ablauf (72) hat, der zur Bereitstellung der alkalisierenden Lösung mit dem Dialysierflüssigkeitskreislauf (5) fluidisch verbindbar, insbesondere verbunden, ist, wobei der Zulauf (68) und der Ablauf (72) der Einsatz-Behälter-Aufnahme (68, 72) vorbestimmt zueinander angeordnet und ausgebildet sind, insbesondere gemäß der vorbestimmten Topologie des Einsatz-Behälters,
**dadurch gekennzeichnet, dass**
eine Behälter-Anordnung (58) gemäß einem der vorhergehenden Ansprüche vorgesehen ist, deren Adapter (66) in die Einsatz-Behälter-Aufnahme (68, 72), vorzugsweise anstatt des Einsatz-Behälters, eingesetzt ist, wobei der Zulauf-Verbindungsabschnitt (70) des Adapters (66) mit dem Zulauf (68) der Einsatz-Behälter-Aufnahme (68, 72) gekoppelt ist und fluidisch verbunden ist, der Ablauf-Verbindungsabschnitt (74) des Adapters (66) mit dem Ablauf (72) der Einsatz-Behälter-Aufnahme (68, 72) zumindest gekoppelt ist und zumindest der Lösungsmittel-Strömungspfad (82) von dem Zulauf-Verbindungsabschnitt (70) des Adapters (66), über den Behälter-Einlauf (62) des Behälters (60), durch den Behälter (60) und zu dem Behälter-Auslauf (64) vorgesehen und ausbildbar, insbesondere ausgebildet, ist.

10. Extrakorporale Blutbehandlungsmaschine (1) nach Anspruch 9, **dadurch gekennzeichnet, dass** eine Erfassungseinheit (76, 78), insbesondere eine Sensoreinheit, vorgesehen und angepasst ist, zu erfassen, ob die Kopplungen ausgebildet sind, oder nicht, und ein von einem Ergebnis der Erfassung abhängiges Signal auszugeben, wobei eine mit der Erfassungseinheit (76, 78) signalverbundene Steuereinheit (54) der Blutbehandlungsmaschine (1) vorgesehen und angepasst ist, eine fluidische Verbindung des Lösungsmittel-Zulaufs (20) mit dem Zulauf (68) der Einsatz-Behälter Aufnahme (68) aufzusteuern, wenn beide Kopplungen als ausgebildet erfasst sind, und zu sperren, wenn nur eine oder keine der Kopplungen als ausgebildet erfasst ist, vorzugsweise durch eine Ansteuerung eines zwischen dem Lösungsmittel-Zulauf (20) und dem Zulauf (68) angeordneten Sperrventils (80).

11. Extrakorporale Blutbehandlungsmaschine (1) nach Anspruch 9 oder 10, **dadurch gekennzeichnet, dass** zur Bereitstellung der alkalisierenden Lösung an den Dialysierflüssigkeitskreislauf (5) ein Bereitstellungs-Strömungspfad (84) von dem Behälter-Auslauf (64) ausgehend und unter Umgehung des Ablauf-Verbindungsabschnitts (74) des Adapters (166) hin zum Dialysierflüssigkeitskreislauf (5) vorgesehen und ausgebildet ist.

12. Extrakorporale Blutbehandlungsmaschine (1) nach Anspruch 9 oder 10, **dadurch gekennzeichnet, dass** zur Bereitstellung der alkalisierenden Lösung an den Dialysierflüssigkeitskreislauf (5) ein Bereitstellungs-Strömungspfad (86) von dem Behälter-Auslauf (64) ausgehend, über den Ablauf-Verbindungsabschnitt (74) des Adapters (66; 266) hin zum Dialysierflüssigkeitskreislauf (5) vorgesehen und ausgebildet ist.
